# FASCICULE DE BREVET EUROPEEN

(11) **EP 2 563 690 B1**
(45) Date de publication et mention de la délivrance du brevet: **23.07.2014**
(21) Numéro de dépôt: 11713825.5
(22) Date de dépôt: 12.04.2011
(51) Int. Cl.: B65D 85/50, A01K 67/033

(54) **CONDITIONNEMENT AMELIORE POUR INSECTES ET/OU ACARIENS AUXILIAIRES**
VERBESSERTE VERPACKUNG FÜR NÜTZLICHE INSEKTEN UND/ODER MILBEN
IMPROVED PACKAGING FOR BENEFICIAL INSECTS AND/OR MITES

(30) Priorité: 20.04.2010 FR 1052987
(43) Date de publication de la demande: 06.03.2013
(73) Titulaire: Biotop, 75016 Paris (FR)
(72) Inventeur: KABIRI, Firouz, F-06100 Nice (FR); FRANDON, Jacques, F-06600 Antibes (FR)
(74) Mandataire: Regimbeau
(86) Numéro de dépôt international: PCT/EP2011/055705
(87) Numéro de publication internationale: WO 2011/131513

(56) Documents cités:
- FR-A1- 2 750 570
- FR-A1- 2 852 937
- US-A- 4 172 336
- US-A- 5 618 220

## Description

La présente invention concerne un conditionnement pour protéger et libérer les insectes et/ou acariens auxiliaires dans le cadre de la lutte biologique (voir FR 285 2937).

### ETAT DE L'ART

Pour utiliser de manière efficace les insectes ou acariens auxiliaires destinés à la lutte biologique contre les ravageurs ou les plantes adventices, il est nécessaire de disposer d'un conditionnement permettant la protection de ces auxiliaires à la fois contre les prédateurs et contre l'environnement extérieur.

Les insectes ou acariens auxiliaires sont typiquement disposés dans un conditionnement à l'état d'adulte, à l'état larvaire, ou à l'état d'oeufs parasités, et peuvent être soit mobiles, soit immobiles.

Les auxiliaires sont préférentiellement choisis selon la période à laquelle ils éclosent, ou plus généralement selon la période ou date à laquelle ils seront aptes à sortir du conditionnement pour remplir leur fonction de prédateurs ou parasitoïdes des ravageurs et/ou antagonistes des plantes adventices. Il est ainsi possible de déterminer la date à laquelle un conditionnement comprenant des auxiliaires doit être utilisé, et disposé dans le milieu souhaité.

L'état de la technique actuel des conditionnements, par exemple le document EP 1606198 présente un conditionnement pour protéger et libérer des insectes et/ou acariens auxiliaires dans le cadre de la lutte biologique, ce conditionnement comprenant un récipient dont les parois présentent un ou plusieurs orifices permettant la sortie des insectes et/ou acariens contenu dans ce récipient.

Les orifices sont disposés de manière à ce que, lorsque les conditionnements sont agencés sous forme de plaques très pratiques pour la production et la manutention, ils ne soient pas ouverts, mais que la séparation des conditionnements ouvre automatiquement ces orifices.

Bien que répondant efficacement aux problématiques de protection des auxiliaires tout en permettant leur diffusion dans le milieu visé, ce conditionnement présente des inconvénients en termes de coûts et de production.

En effet, ce conditionnement est typiquement réalisé en carton plein, et il est par conséquent nécessaire d'y aménager les ouvertures permettant la sortie des auxiliaires vers le milieu visé. Or, la réalisation d'ouvertures dans du carton plein nécessite un outillage onéreux, adapté à des pressions élevées pour la découpe, et d'une grande précision du fait de la petitesse des ouvertures de sortie.

La quantité de matière première nécessaire pour la réalisation des conditionnements selon l'état de la technique est par ailleurs importante, ce qui entraîne un coût élevé et une masse importante de ces conditionnements.

La présente invention vise donc à présenter un conditionnement pour protéger et libérer les insectes et/ou acariens auxiliaires, dont la réalisation est facilitée et dont le coût est réduit par rapport aux conditionnements selon l'état de l'art.

### PRESENTATION DE L'INVENTION

La présente invention concerne une plaque pour protéger et libérer des insectes et/ou acariens auxiliaires, comprenant les caractéristiques de la revendication 1.

Cette description concerne également une plaque comprenant au moins un conditionnement tel que défini précédemment, ladite plaque comprenant des bandes latérales compressées s'étendant sur au moins deux côtés opposés de ladite plaque, de manière à obstruer les ouvertures définies par la couche intermédiaire dudit au moins un conditionnement.

En outre, cette description présente un procédé de fabrication d'un conditionnement pour protéger et libérer des insectes et/ou acariens auxiliaires, comprenant les étapes suivantes :
- découpe d'une feuille de matériau cannelé à l'aide d'un emporte pièce de manière à réaliser un perçage débouchant sur toute l'épaisseur de ladite feuille de matériau cannelé, ledit perçage définissant un espace interne ayant une bordure s'étendant sur toute sa périphérie,
- collage d'une couche inférieure contre une première face de la feuille de matériau cannelé,
- introduction d'auxiliaires dans l'espace interne,
- collage d'une couche supérieure contre la seconde face de la feuille de matériau cannelé.

### PRESENTATION DES FIGURES

D'autres caractéristiques, buts et avantages de l'invention ressortiront de la description qui suit, qui est purement illustrative et non limitative, et qui doit être lue en regard des dessins annexés, sur lesquels :
- la figure 1 présente une vue d'un conditionnement amélioré,
- les figures 2, 3 et 4 présentent plusieurs vues détaillées de ce conditionnement amélioré,
- la figure 5 présente une plaque composée de plusieurs conditionnements améliorés,
- les figures 6, 7 et 8 présentent plusieurs vues d'un mode de réalisation particulier de ce conditionnement amélioré,
- les figures 9, 10 et 11 présentent plusieurs vues d'un autre mode de réalisation particulier de ce conditionnement amélioré,
- les figures 12a, 12b et 12c présentent plusieurs modes de réalisation de cannelures.

### DESCRIPTION DETAILLEE

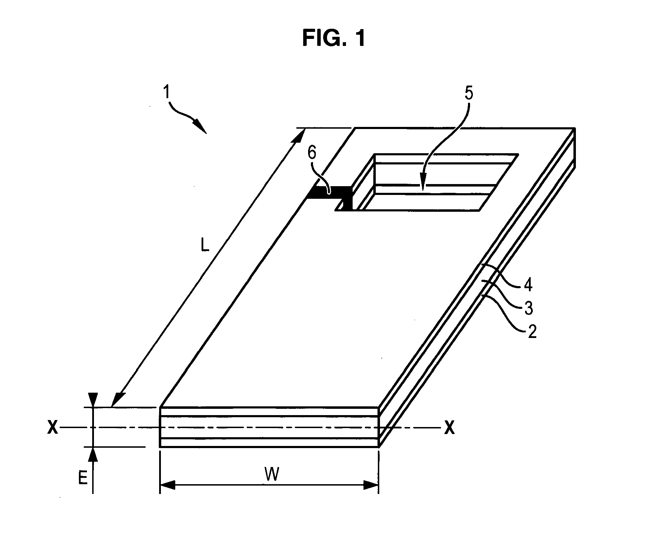

La figure 1 présente un mode de réalisation d'un conditionnement amélioré 1 pour protéger et libérer des insectes et/ou acariens auxiliaires.

Ce conditionnement 1 a sensiblement une forme de parallélépipède rectangle définissant ainsi une épaisseur E, une longueur L et une largeur W, et comprend une couche inférieure 2, une couche intermédiaire 3 et une couche supérieure 4, ces couches étant superposées et assemblées dans cet ordre.

Le conditionnement 1 n'est pas limité à une forme de parallélépipède rectangle, et peut prendre toute forme adaptée.

Il comprend en outre un dispositif d'accrochage sous la forme d'une languette 5, ayant ici la forme d'un perçage de forme sensiblement rectangulaire traversant, mais pouvant prendre toute autre forme adaptée, notamment rond ou carré, tout en conservant sa fonction de dispositif d'accrochage. Cette languette 5 peut être associée à une prédécoupe 6 pouvant également prendre la forme d'une rainure, permettant d'ouvrir le perçage qui se présente alors sous la forme d'un crochet ouvert, par exemple pour positionner le conditionnement 1 sur un fil, ou plus généralement pour mettre le conditionnement 1 en position dans le milieu visé.

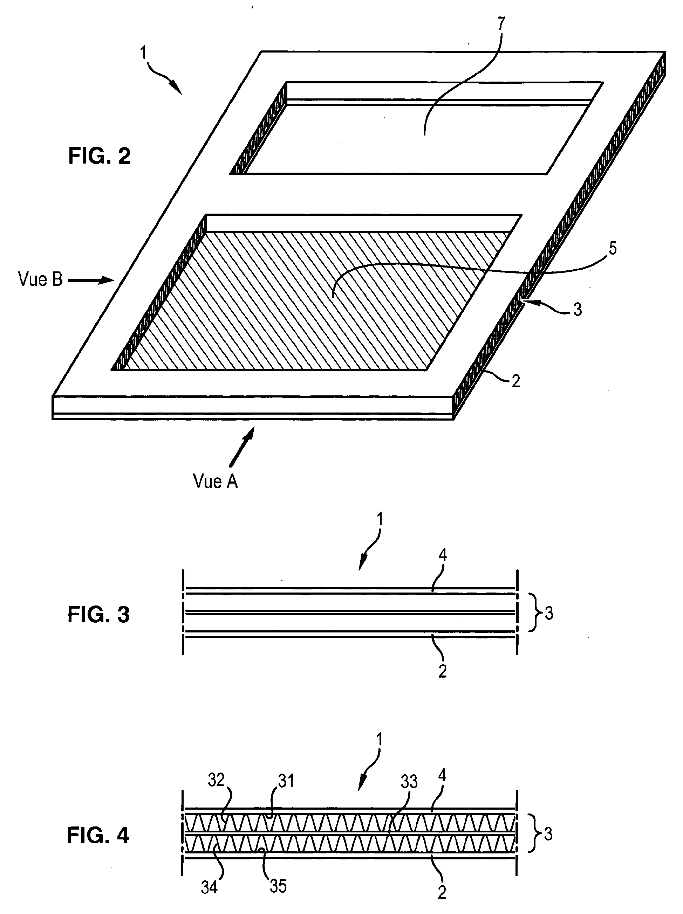

La Figure 2 présente une vue du conditionnement 1 selon le plan de coupe X-X défini sur les figures 1, 3 et 4, et permettant ainsi d'observer la structure interne du conditionnement 1.

Le conditionnement 1 comprend un espace interne 7, aménagé dans la couche intermédiaire 3.

A la vue des figures 1 et 2, on comprend que l'espace interne 7 est délimité par une surface de la couche inférieure 2, une surface de la couche supérieure 4, et sur toute sa périphérie par la couche intermédiaire 3 ; l'espace interne 7 n'étant pas communiquant avec la languette 5 en raison du sens des cannelures du matériau parallèles à la largeur du conditionnement.

Tel que représenté sur la figure 2, la couche intermédiaire 3 est réalisée en matériau cannelé présentant des cannelures.

L'utilisation de matériau cannelé pour la couche intermédiaire 3 permet de définir des ouvertures de communication entre l'espace interne 7 du conditionnement 1 et l'extérieur de ce conditionnement 1 ; les cannelures définissent en effet des galeries selon leur longueur.

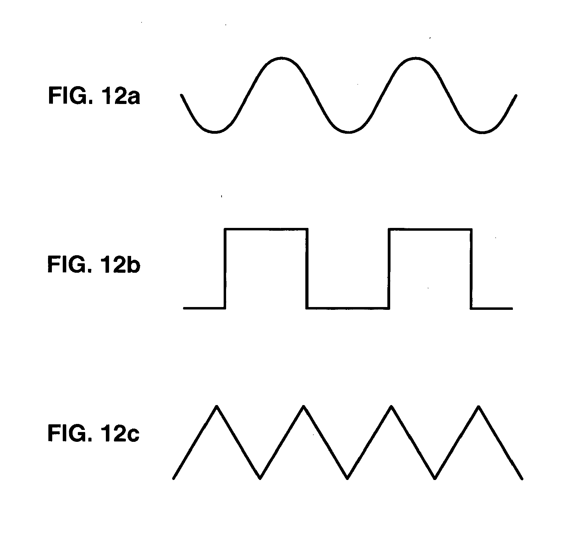

Par matériau cannelé, on entend tout matériau présentant des cannelures, à savoir des rainures ou sillons de forme régulière. Les figures 12a, 12b et 12c présentent des vues en coupe de plusieurs modes de réalisation de cannelures, respectivement des cannelures de forme ondulatoire, des cannelures en créneau et des cannelures en triangle. On comprendra que d'autres formes de cannelures sont possibles, tout en conservant des rainures ou sillons réguliers ou sensiblement réguliers de manière à permettre la communication entre l'espace interne 7 et l'extérieur du conditionnement 1.

Dans le conditionnement illustré sur la figure 2, les cannelures de la couche intermédiaire 3 sont sensiblement parallèles entre elles, et sont disposées selon la largeur du conditionnement 1, elles définissent donc les ouvertures permettant la communication entre l'espace interne 7 du conditionnement 1 et l'extérieur selon la largeur W du conditionnement 1.

De plus, du fait de leur disposition dans le sens de la largeur W du conditionnement 1, les cannelures assurent qu'aucune communication ne peut se produire dans le sens de la longueur L du conditionnement 1, ce qui assure en même temps une protection des produits conditionnés dans l'espace interne 7 contre la pluie et l'irrigation dans le cas ou le conditionnement est utilisé en extérieur et accroché aux plantes par son dispositif d'accrochage ou languette 5, de manière à ce que les cannelures soient alignées horizontalement.

Cet aspect est mis en avant sur les figures 3 et 4, qui illustrent le conditionnement 1 selon les vues A et B définies sur la figure 2.

La figure 3 met en avant l'effet des cannelures dans la couche intermédiaire 3 ; celles-ci vont en effet obstruer le passage selon la longueur L du conditionnement 1.

La figure, 4 met en avant l'effet inverse, à savoir les galeries définies par les cannelures et s'étendant sur toute la largeur W du conditionnement 1 et permettant la communication entre l'espace interne 7 du conditionnement 1 et l'extérieur.

Tel qu'illustré, la couche intermédiaire 3 présente une cannelure double ; elle se compose alors d'une alternance de feuilles planes et de feuilles cannelées d'un matériau donné ;
- une première feuille plane supérieure 31,
- une première feuille cannelée 32,
- une première feuille plane inférieure 33
- une seconde feuille plane supérieure 34
- une seconde feuille cannelée 35,
- une seconde feuille plane inférieure 36,
ces feuilles étant superposées dans cet ordre.

D'autres modes de réalisation sont possibles ; la couche intermédiaire 3 peut par exemple être à cannelure simple, voire à cannelure triple ou plus, selon l'épaisseur souhaitée et la taille des ouvertures souhaitées.

La couche intermédiaire 3 peut également n'être composée que d'une unique feuille cannelée, les couches supérieures 4 et inférieure 2 permettant alors d'assurer la rigidité de l'ensemble, en remplissant la fonction des feuilles planes typiquement utilisées pour le matériau cannelé.

En variante, les couches inférieure 2 et supérieure 4 peuvent permettre de s'affranchir d'une partie des feuilles planes supérieures et/ou inférieures.

Plus précisément, en reprenant le mode de réalisation illustré sur les figures 3 et 4, il possible de s'affranchir de la première feuille plane supérieure 31 et de la seconde feuille plane inférieure 36, les feuilles cannelées 32 et 35 étant alors assemblées directement sur les couches supérieure 4 et inférieure 2. La première feuille plane inférieure 33 et la seconde feuille plane supérieure 34 permettent quant à elles de faciliter l'assemblage, en présentant des surfaces planes.

Cette variante peut également s'appliquer à d'autres modes de réalisation, par exemple en cas de couche intermédiaire 3 à cannelure simple. Le conditionnement 1 comprend alors typiquement une couche inférieure 2, sur laquelle est directement disposée une feuille cannelée, laquelle est surmontée d'une feuille plane qui va faciliter l'assemblage de la couche supérieure 4 sur cette dernière.

De manière générale, de nombreuses variantes de matériaux cannelés peuvent être utilisées, ces variantes étant bien connues de l'Homme du métier.

Selon un mode de réalisation particulier, la surface de la couche inférieure 2 et/ou la surface de la couche supérieure 4 qui délimitent l'espace interne 7 comprennent des moyens de collage, par exemple une surface adhésive, adaptés pour maintenir en position les auxiliaires, typiquement lorsqu'ils sont sous forme larvaire ou d'oeufs, ainsi qu'éventuellement la nourriture associée auxdits auxiliaires.

Ces moyens de collage permettent le maintien en position d'oeufs ou de larves d'insectes et/ou acariens auxiliaires disposés dans le conditionnement 1, et permettent de s'assurer que les oeufs ou larves d'insectes et/ou acariens auxiliaires contenus dans le conditionnement 1 ne s'échappent pas de l'espace interne 7 par les ouvertures définies par les cannelures du matériau cannelé.

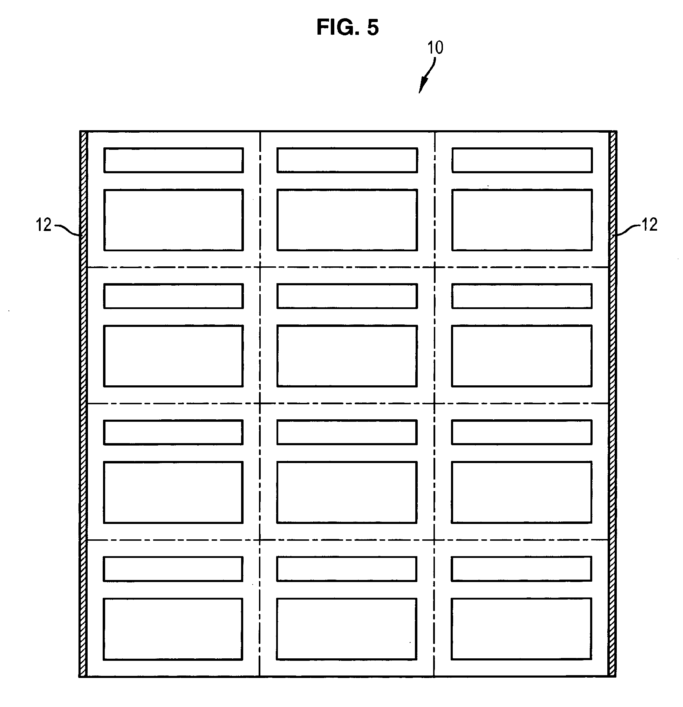

La figure 5 présente une plaque 10 comprenant des conditionnements similaires au conditionnement présenté sur les figures 1 à 4.

La plaque 10 présente douze conditionnements, répartis en trois colonnes et quatre lignes, tous les conditionnements étant disposés selon la même orientation.

A la lecture de ce qui précède, on comprend que la communication entre les espaces internes de conditionnements d'une même colonne n'est pas possible, du fait de l'orientation des cannelures dans le sens des lignes.

Selon un mode de réalisation particulier, la plaque 10 présente une bande comprimée 12 à ses deux extrémités présentant des ouvertures du fait de l'orientation des cannelures, empêchant ainsi les auxiliaires présents dans les espaces internes de s'échapper de la plaque 10.

Cette bande comprimée 12 a typiquement une largeur de l'ordre de 3 à 6 mm.

Les conditionnements sont séparés par des lignes de découpe horizontales 14 et verticales 15, qui vont permettre à l'utilisateur de séparer les conditionnements de la plaque 10.

Lors de l'utilisation, l'utilisateur peut alors également retirer les bandes comprimées 12, de manière à ne plus obstruer les ouvertures des conditionnements initialement disposés sur les côtés de la plaque 10.

Dans le cas d'une plaque comprenant plusieurs conditionnements sur une même ligne et lorsque les cannelures sont orientées dans le sens des lignes, il n'est alors pas indispensable de retirer les bandes comprimées 12, dans la mesure où les ouvertures d'au moins un côté des conditionnements ne seront pas obturées.

L'éventuel retrait de la ou des bande(s) comprimée(s) 12 peut être effectué soit par découpage, soit via la réalisation de prédécoupes dès la fabrication de la plaque 10.

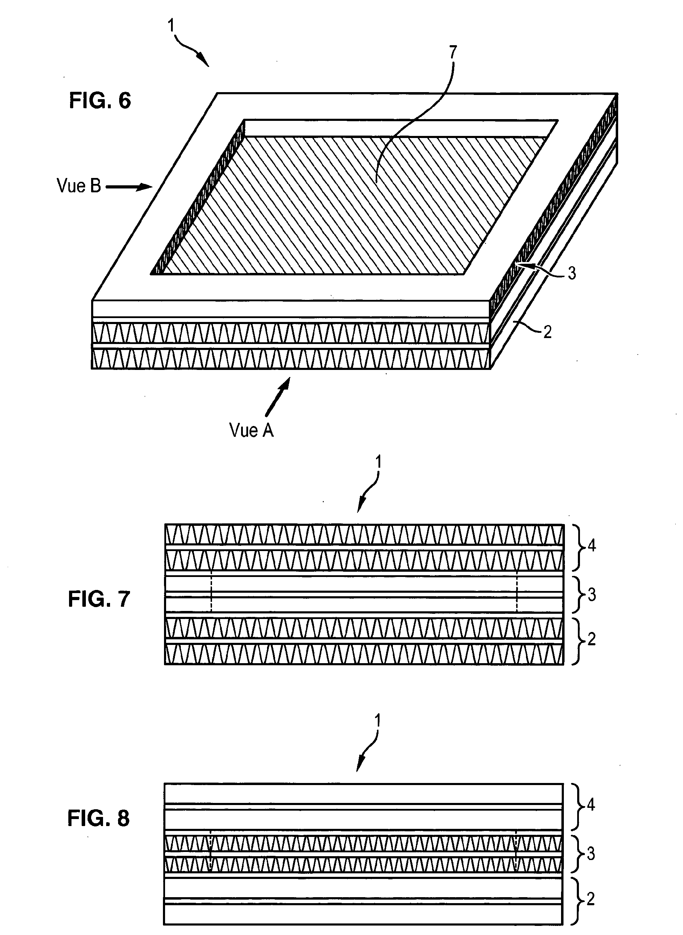

Les figures 6 à 11 présentent plusieurs vues d'autres modes de réalisation d'un conditionnement amélioré.

Les figures 6, 7 et 8 sont les vues équivalentes de celles présentées sur les figures 2, 3 et 4 pour un premier mode de réalisation alternatif.

Dans ce premier mode de réalisation alternatif, la couche inférieure 2 et la couche supérieure 4 sont composées de matériau cannelé à double cannelure, tout comme la couche intermédiaire 3.

Des variantes sont bien entendu possibles, dans lesquelles les différentes couches 2, 3 et 4 sont à simple ou triple cannelure, voire plus.

Tel qu'illustré, les cannelures de chacune des couches sont parallèles entre elles, et les cannelures de la couche inférieure 2, de la couche intermédiaire 3 et de la couche supérieure 4 sont orientées de manière alternée, les cannelures de la couche intermédiaire 3 étant orientées de manière sensiblement perpendiculaire aux cannelures de la couche inférieure 2 et de la couche supérieure 4.

Ce mode de réalisation avantageux permet de rigidifier le conditionnement 1 par rapport à une variante de ce mode de réalisation présentant des cannelures toutes orientées dans la même direction, dans le cas où une telle rigidité du conditionnement est requise.

Tel que représenté, ce mode de réalisation ne présente pas de languette 5 semblable à celle illustrée sur les figures 1 à 5. En effet, ce mode de réalisation est typiquement destiné à être utilisé pour épandage mécanique au sol, et ne nécessite donc pas de moyens d'accrochage.

L'utilisation de couches supérieure 4 et inférieure 2 comprenant un matériau cannelé permet d'augmenter considérablement l'épaisseur de ces couches supérieure 4 et inférieure 2 tout en conservant une masse réduite, de manière à séparer l'espace interne 7 du sol sur lequel le conditionnement 1 est épandu.

Les auxiliaires contenus dans l'espace interne 7 sont ainsi mieux protégés de l'humidité excessive en cas de pluie ou d'irrigation et de la chaleur excessive en cas de sol directement exposé au soleil par rapport au cas où les couches supérieure 4 et inférieure 2 seraient plus fines, composées d'un matériau plein ne présentant pas ces cannelures.

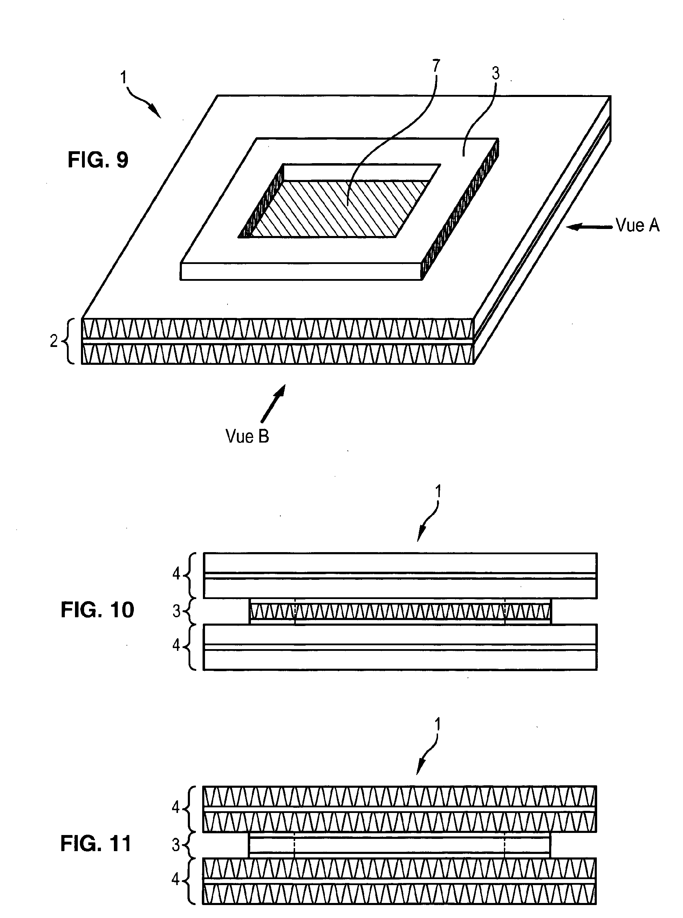

Les figures 9, 10 et 11 sont les vues sensiblement équivalentes de celles présentées sur les figures 2, 3 et 4 pour un second mode de réalisation alternatif.

Ce second mode de réalisation alternatif présente également une couche supérieure 4 et une couche inférieure 2 composées d'un matériau à double cannelure, la couche intermédiaire étant quant à elle composée d'un matériau a cannelure simple.

Par rapport au mode de réalisation présenté sur les figures 6 à 8, on observe que la couche intermédiaire 3 a ici une surface inférieure à celle des couches supérieure 4 et inférieure 2.

Ce mode de réalisation permet d'accroître la protection des auxiliaires contenus dans l'espace interne 7 en augmentant la distance séparant ledit espace interne 7 du sol sur lequel le conditionnement 1 est épandu, et ce quelle que soit la disposition du conditionnement 1 (sur une des couches supérieure 4 ou inférieure 2 ou sur l'une des tranches du conditionnement 1).

Les variantes illustrées des différents modes de réalisation sont purement illustratives. D'autres variantes sont bien entendu envisageables, présentant par exemple des couches inférieure 2, intermédiaire 3 et supérieure 4 d'épaisseur variable et de nombre de cannelures variable ; typiquement cannelure simple, double, triple ou plus.

Les dimensions de l'espace interne 7 ainsi que le matériau cannelé utilisé sont choisis de manière à être adaptés à la taille des auxiliaires.

A titre d'exemple :
- pour un conditionnement de 1000 à 2000 Trichogrammes, l'espace interne 7 a typiquement une surface comprise entre 3 et 30 cm², une épaisseur de l'ordre de 2.2 mm, et les cannelures définissent des ouvertures dont la dimension est de l'ordre de 1 mm ;
- pour un conditionnement de 500 à 3000 acariens, l'espace interne 7 a typiquement une surface comprise entre 15 et 40 cm², une épaisseur de l'ordre de 3 mm, et les cannelures définissent des ouvertures dont la dimension est de l'ordre de 1 mm ;
- pour un conditionnement de 50 à 200 Orius et Anthocoris, l'espace interne 7 a typiquement une surface comprise entre 50 et 100 cm², une épaisseur de l'ordre de 10 mm, et les cannelures définissent des ouvertures dont la dimension est de l'ordre de 4*2 mm ;
- pour un conditionnement de 50 à 200 Macrolophus, l'espace interne 7 a typiquement une surface comprise entre 50 et 100 cm², une épaisseur de l'ordre de 15 mm, et les cannelures définissent des ouvertures dont la dimension est de l'ordre de 5*3 mm ;
- pour un conditionnement de 50 à 100 parasites de pucerons, l'espace interne 7 a typiquement une surface comprise entre 20 et 50 cm², une épaisseur de l'ordre de 5 mm, et les cannelures définissent des ouvertures dont la dimension est de l'ordre de 2*2 mm.

La taille des ouvertures résultant de l'utilisation de matériau cannelé peut par ailleurs être modifiée en exerçant une compression sur ledit matériau cannelé, de manière à adapter les ouvertures aux auxiliaires que l'on souhaite disposer dans l'espace interne 7.

Par ailleurs, un grand nombre d'ouvertures entre l'espace interne 7 et l'extérieur du conditionnement 1 est défini du fait de l'utilisation de matériau cannelé, permettant ainsi de réduire les risques d'obstruction des ouvertures, par exemple en cas d'entassement des auxiliaires dans une même ouverture, ou en cas d'introduction d'éléments extérieurs.

L'utilisation de matériau cannelé permet un gain de matière important, qui va notamment influer sur le poids du conditionnement et sur son coût. Ce gain de poids va être avantageux, notamment pour toutes les étapes logistiques.

Par exemple, un conditionnement pour Trichogrammes réalisé en carton plein d'une épaisseur de 1.4 mm a un poids de l'ordre de 1400 g/m², tandis qu'on conditionnement amélioré pour Trichogrammes tel que présenté réalisé en carton ondulé aura un poids de l'ordre de 240 g/m².

Le conditionnement amélioré a donc un poids considérablement réduit par rapport aux conditionnements selon l'état de l'art, permettant de réaliser une économie de matière première importante (plus de 80% pour l'exemple cité ci-dessus).

D'autres matériaux que le carton ou le carton ondulé peuvent être utilisés, par exemple la cellulose moulée, le papier, ou plus généralement les matériaux composés de fibres végétales, ou encore le plastique. Les différentes couches 2, 3 et 4 peuvent être réalisées dans des matériaux identiques ou différents. L'utilisation des matériaux biodégradables tels que le carton ondulé, le papier ou le plastique biodégradable présente toutefois un intérêt certain compte tenu des applications de ce conditionnement 1.

La réalisation d'un conditionnement 1 amélioré tel que présenté comprend typiquement les étapes suivantes :
a/ découpe d'une feuille de matériau cannelé constituant la couche intermédiaire 3 à l'aide d'un emporte pièce de manière à réaliser un ou plusieurs perçage(s) débouchant(s) sur toute l'épaisseur de ladite feuille de matériau cannelé, ledit perçage définissant l'espace interne 7 ;
b/ collage de la couche inférieure 2 contre une première face de la couche intermédiaire 3;
c/ le cas échéant, découpe du conditionnement 1 à l'aide d'un emporte pièce de manière à former la languette 5 ainsi qu'éventuellement la prédécoupe 6 ;
d/ introduction d'auxiliaires dans l'espace interne 7 de la couche intermédiaire 3 ;
e/ collage d'une couche supérieure 4 contre la seconde face de la couche intermédiaire 3, la superposition de la couche inférieure 2, la couche intermédiaire 3 et la couche supérieure 4 formant alors le conditionnement 1.

Ce procédé de réalisation d'un conditionnement 1 peut être adapté aux différents modes de réalisation précédemment, notamment en ce qui concerne les variantes de structure des couches supérieure 4, intermédiaire 3 et inférieure 2.

Selon une alternative, l'étape de découpe du conditionnement 1 à l'aide d'un emporte pièce de manière à former la languette 5 ainsi que le cas échéant, la prédécoupe 6 peut intervenir à la fin de la fabrication suite à l'introduction des auxiliaires.

Le conditionnement 1 tel que présenté permet donc de répondre aux mêmes exigences que les conditionnements selon l'état de l'art en termes de maintien et de protection des auxiliaires, tout en présentant une structure plus simple et plus économique, utilisant une quantité moindre de matière première et ne nécessitant pas de perçages spécifiques de manière à réaliser des ouvertures entre l'espace interne 7 du conditionnement 1 et l'extérieur, ces ouvertures résultant de l'utilisation du matériau cannelé.

## Revendications

1. Plaque (10) comprenant une pluralité de conditionnements (1) pour protéger et libérer des insectes et/ou acariens auxiliaires, chacun desdits conditionnements comprenant :
- une couche supérieure (4),
- une couche intermédiaire (3),
- une couche inférieure (2),
**caractérisée en ce que** les couches (2, 3, 4) sont superposées et assemblées dans cet ordre, chacun des conditionnements (1) comprenant un espace interne (7) aménagé dans sa couche intermédiaire (3), délimité de toutes parts par des parois desdites couches (2, 3, 4), la couche intermédiaire (3) comprenant au moins une feuille de matériau cannelé dont les cannelures définissent des ouvertures de communication entre l'espace interne (7) du conditionnement (1) et l'extérieur,
la plaque (10) comprenant des bandes latérales compressées (12) s'étendant sur au moins deux côtés opposés de la plaque (10), de manière à obstruer les ouvertures définies par la couche intermédiaire (3) des conditionnement (1) formant la plaque (10).

2. Plaque (10) selon la revendication précédente, dans laquelle la couche supérieure (4) et la couche inférieure (2) sont réalisées en matériau cannelé.

3. Plaque (10) selon la revendication 2, dans laquelle les cannelures de chacune desdites couches (2, 3, 4) sont sensiblement parallèles entre elles, et en ce que les cannelures des couches supérieure (4) et inférieure (2) sont orientées de manière sensiblement perpendiculaire aux cannelures de la couche intermédiaire (3).

4. Plaque (10) selon l'une des revendications 1 à 3, dans laquelle ladite couche intermédiaire (3) a une surface inférieure à la surface des couches supérieure (4) et inférieure (2).

5. Plaque (10) selon l'une des revendications 1 à 4, dans laquelle ledit matériau cannelé est du plastique, ou un matériau composé de fibres végétales tel que du carton, du carton ondulé, du papier ou de la cellulose moulée.

6. Plaque (10) selon l'une des revendications 1 à 5, dans laquelle au moins une des parois desdites couches (2, 3, 4) délimitant l'espace interne (7) comprend des moyens de collage.

7. Plaque (10) selon l'une des revendications 1 à 6, dans laquelle chacun des conditionnements (1) comprend en outre une languette (5) traversante.

8. Plaque (10) selon la revendication 7, dans laquelle chacun des conditionnements (1) a une forme sensiblement rectangulaire, et que les cannelures de la couche intermédiaire (3) sont disposées de manière sensiblement parallèle, dans le sens de la largeur (W) dudit conditionnement (1), ledit espace interne (7) et ladite languette (5) étant disposées dans le sens de la longueur (L) dudit conditionnement (1).

## Patentansprüche

1. Platte, umfassend eine Vielzahl von Verpackungen (1) zum Schützen und Freisetzen von nützlichen Insekten und/oder Milben, wobei jede der Verpackungen Folgendes umfasst:
- eine obere Schicht (4),
- eine mittlere Schicht (3),
- eine untere Schicht (2),
**dadurch gekennzeichnet, dass** die Schichten (2, 3, 4) in dieser Reihenfolge übereinander gelegt und verbunden sind, wobei jede der Verpackungen (1) einen inneren Raum (7) umfasst, der in ihrer mittleren Schicht (3) angeordnet ist und auf allen Seiten durch Wände der Schichten (2, 3, 4) begrenzt ist, wobei die mittlere Schicht (3) mindestens eine Lage aus geriffeltem Material umfasst, deren Riffelungen Verbindungsöffnungen zwischen dem inneren Raum (7) der Verpackung (1) und der Außenumgebung definieren,
wobei die Platte (10) zusammengedrückte seitliche Streifen (12) umfasst, die sich auf mindestens zwei entgegengesetzten Seiten der Platte (10) erstrecken, derart, dass sie die Öffnungen versperren, die durch die mittlere Schicht (3) der Verpackungen (1), welche die Platte (10) bilden, definiert werden.

2. Platte (10) nach dem vorhergehenden Anspruch, wobei die obere Schicht (4) und die untere Schicht (2) aus geriffeltem Material gebildet sind.

3. Platte (10) nach Anspruch 2, wobei die Riffelungen jeder der Schichten (2, 3, 4) im Wesentlichen zueinander parallel sind und dass die Riffelungen der oberen (4) und der unteren (2) Schicht zu den Riffelungen der mittleren Schicht (3) im Wesentlichen senkrecht ausgerichtet sind.

4. Platte (10) nach einem der Ansprüche 1 bis 3, wobei die mittlere Schicht (3) eine Oberfläche hat, die kleiner ist als die Oberfläche der oberen (4) und der unteren (2) Schicht.

5. Platte (10) nach einem der Ansprüche 1 bis 4, wobei das geriffelte Material Kunststoff oder ein Verbundmaterial aus Pflanzenfasern ist, wie Pappe, Wellpappe, Papier oder Formzellulose.

6. Platte (10) nach einem der Ansprüche 1 bis 5, wobei mindestens eine der Wände der Schichten (2, 3, 4), die den inneren Raum (7) begrenzen, Klebemittel enthält.

7. Platte (14) nach einem der Ansprüche 1 bis 6, wobei jede der Verpackungen (1) ferner eine durchgehende Zunge (5) umfasst.

8. Platte (10) nach Anspruch 7, wobei jede der Verpackungen (1) eine im Wesentlichen rechteckige Form hat und dass die Riffelungen der mittleren Schicht (3), in der Richtung der Breite (W) der Verpackung (1), im Wesentlichen parallel angeordnet sind, wobei der innere Raum (7) und die Zunge (5) in der Richtung der Länge (L) der Verpackung (1) angeordnet sind.

## Claims

1. A plate (10) comprising a plurality of packaging units (1) for protecting and releasing auxiliary insects and/or mites, each of said packaging units comprising:
- an upper layer (4),
- an intermediate layer (3),
- a lower layer (2),
**characterized in that** the layers (2, 3, 4) are superposed and assembled in this order, each of the packaging units (1) comprising an internal space (7) laid out in its intermediate layer (3), delimited on all sides by walls of said layers (2, 3, 4), the intermediate layer (3) comprising at least one fluted material sheet, the flutes of which define apertures for communication between the internal space (7) of the packaging unit (1) and the outside,
the plate (10) comprising compressed side bands (12) extending over at least the two opposite sides of the plate (10), so as to block the apertures defined by the intermediate layer (3) of the packaging units (1) forming the plate (10).

2. The plate (10) according to the preceding claim, wherein the upper layer (4) and the lower layer (2) are made in a fluted material.

3. The plate (10) according to claim 2, wherein the flutes of each of said layers (2, 3, 4) are substantially parallel with each other, and in that the flutes of the upper (4) and lower (2) layers are oriented substantially perpendicularly to the flutes of the intermediate layer (3).

4. The plate (10) according to one of claims 1 to 3, wherein said intermediate layer (3) has a surface area of less than the surface area of the upper (4) and lower (2) layers.

5. The plate (10) according to one of claims 1 to 4, wherein the fluted material is plastic, or a material consisting of plant fibers such as cardboard, corrugated cardboard, paper or molded cellulose.

6. The plate (10) according to one of claims 1 to 5, wherein at least one of the walls of said layers (2, 3, 4) delimiting the internal space (7) comprises adhesive bonding means.

7. The plate (10) according to one of claims 1 to 6, wherein each of the packaging units (1) further comprises a through-tab (5).

8. The plate (10) according to claim 7, wherein, each of the packaging units (1) has a substantially rectangular shape, and in that the flutes of the intermediate layer (3) are positioned substantially in parallel, in the direction of the width (W) of said packaging unit (1), said internal space (7) and said tab (5) being positioned in the direction of the length (L) of said packaging unit (1).
